# EUROPÄISCHE PATENTANMELDUNG

(11) **EP 0 689 846 A1**
(43) Veröffentlichungstag der Anmeldung: **03.01.1996**
(21) Anmeldenummer: 95107957.3
(22) Anmeldetag: 25.05.1995
(51) Int. Cl.: A61M 1/02

(54) **Vorrichtung und Verfahren zum Mischen von Antikoagulans mit Frischblut**

(30) Priorität: 27.06.1994 DE 9410237 U
(71) Anmelder: NPBI Nederlands Produktielaboratorium voor Bloedtransfusieapparatuur en Infusievloeistoffen B.V., NL-7881 HM Emmer Compascuum (NL)
(72) Erfinder: Biekart, Frank Th., NL-7822 BP Emmen (NL); Hilbrink, Hubertus E., NL-7827 BP Emmen (NL)
(74) Vertreter: Masch, Karl Gerhard, Dr.

(57) **Zusammenfassung**

Zum Mischen von in einem Blutbeutel befindlichem flüssigen Antikoagulans mit während einer Blutentnahme in den Blutbeutel einlaufendem Frischblut, ist eine von einem Antrieb in Bewegung versetzbare flache Aufnahmeschale (1) für den Blutbeutel vorgesehen. Für einwandfreie Durchmischung ist die horizontal angeordnete Aufnahmeschale (1) auf zwei mit vorgegebenem Abstand voneinander angeordneten Vertikalzapfen (3, 4) drehbar gelagert und sind die beiden Vertikalzapfen (3, 4) mit derselben Exzentrizität auf jeweils einer um eine vertikale Achse (7, 8) drehbar gelagerten Exzenterscheibe (9, 10) befestigt; zumindest eine der beiden Exzenterscheiben (9, 10) ist vom Antrieb (2) in Drehung versetzbar. Auf diese Weise wird der Blutbeutel horizontal in eine kreisförmige Taumelbewegung versetzt.

## Beschreibung

Die Erfindung betrifft eine Vorrichtung und ein Verfahren zum Mischen von in einem Blutbeutel befindlichem flüssigen Antikoagulans mit während einer Blutentnahme in den Blutbeutel einlaufendem Frischblut, bestehend aus einer von einem Antrieb in Bewegung versetzbaren flachen Aufnahmeschale für den Blutbeutel.

Blut wird von Hospitälern, Blutbanken und anderen medizinischen Einrichtungen für die Verwendung bei Bluttranfusionen in Vorrat gehalten. Das Frischblut wird von einzelnen Spendern in den Hospitälern, Blutbanken oder an anderen Stellen gesammelt. Wichtig ist, daß die Qualität der Blutvorräte in der Zeit zwischen Entnahme und Benutzung bei der Transfusion aufrechterhalten bleibt. Hierzu wird das gesammelte Frischblut mit einem Antikoagulans vermischt, um das koagulieren des Blutes zu verhindern. Gesamtblut bzw. Frischblut wird in geschlossenen Mehrfachblutbehältersystemen aus Kunststoff gesammelt. Der erste Behälter solcher Systeme, der Aufnahmebeutel enthält das flüssige Antikoagulans, um das gespendete Blut am Klumpen zu hindern. Während der Blutentnahme muß der Frischblutstrom hinreichend mit dem Antikoagulans vermischt werden, um eine homogene Mischung zu ergeben.

Um diesen Zweck zu erreichen, ist es zunächst bekannt, den das Frischblut aufnehmenden Blutbeutel von Hand am Beginn der Entnahmeprozedur zu kneten. Das ist für den Spender angenehm, da er in sozialem Kontakt mit einem menschlichen Wesen während der Blutspende ist. Das manuelle Kneten hat aber den Nachteil, daß man nicht unmittelbar sehen kann, ob das Mischen in ausreichendem Maße erfolgt ist. Aus diesem Grunde sind auch entsprechende Vorrichtungen bekannt, die das Mischen automatisch vornehmen.

Bei einer aus der Praxis bekannten, druckschriftlich nicht näher belegten Vorrichtung der eingangs genannten Art wird die Aufnahmeschale vom Antrieb in eine Schaukelbewegung versetzt. Untersuchungen haben aber ergeben, daß der Mischeffekt unzureichend ist. Aufgrund der Dichteunterschiede des flüssigen Antikoagulans und des Frischblutes kann es nämlich insbesondere anfänglich zu Überschichtungen kommen, die beim weiteren Zufließen von Frischblut nicht mehr vollständig miteinander beseitigbar sind. Folglich beobachtet man auch in solchen Fällen Klumpeffekte im Frischblut.

Der Erfindung liegt die Aufgabe zugrunde, die Maßnahmen der eingangs genannten Art so weiter zu entwickeln, daß eine einwandfreie Durchmischung von flüssigem Antikoagulans und Frischblut bei der Blutentnahme sichergestellt ist.

Die erfindungsgemäße Lösung dieser Aufgabe besteht in vorrichtungsmäßiger Hinsicht darin, daß die horizontal angeordnete Aufnahmeschale auf zwei mit vorgegebenem Abstand voneinander angeordneten Vertikalzapfen drehbar gelagert ist, daß die beiden Vertikalzapfen mit derselben Exzentrizität auf jeweils einer um eine vertikale Achse drehbar gelagerten Exzenterscheibe befestigt sind und daß zumindest eine der beiden Exzenterscheiben vom Antrieb in der Drehung versetzbar ist.

Die Erfindung geht hierbei von der überraschenden Erkenntnis aus, daß dann, wenn der Blutbeutel mit dem flüssigen Antikoagulans und dem zufließenden Frischblut in horizontal bleibender Lage in eine in dieser Horizontalebene stattfindende kreisförmige Taumelbewegung versetzt wird, in jedem Fall eine einwandfreie Durchmischung der beiden Flüssigkeiten stattfindet, die jegliche Klumpenbildung verhindert. Dieses Ergebnis war aufgrund bisheriger Erkenntnisse nicht zu erwarten.

Nach bevorzugter Ausführungsform der Erfindung besteht der Antrieb aus einem Elektromotor mit Reibrad. Im einzelnen können dann die beiden, denselben Durchmesser aufweisenden Exzenterscheiben von dem zwischen diesen angeordneten Reibrad angetrieben sein.

Im Rahmen der Erfindung kommt den beiden folgenden Maßnahmen besondere Bedeutung zu, die folglich bevorzugt verwirklicht werden: Die Exzentrizität des Vertikalzapfens gegenüber der Drehachse der zugeordneten Exzenterscheibe soll wenigstens 20 mm ausmachen. Außerdem sollen die Exzenterscheiben mit wenigstens einer Umdrehung pro Sekunde angetrieben sein.

Bei den bekannten Vorrichungen wird gleichzeitig beim Mischen eine Wiegung durchgeführt, damit festgestellt werden kann, wenn der Blutbeutel mehr oder weniger voll ist. Bei der bekannten Vorrichtung ist es hierzu aber erforderlich, daß die hin- und hergehende Taumelbewegung unterbrochen wird und die Aufnahmeschale in eine horizontale Lage verbracht wird. Im Rahmen der erfindungsgemäßen Maßnahmen ist eine solche Wiegung wesentlich einfacher möglich, indem nämlich der Antrieb und die beiden Exzenterscheiben an einer z.B. plattenförmigen Lageranordnung vorgesehen sind, die auf Drucksensoren einer Wiegeeinrichtung aufgelagert ist. Bei einer solchen Ausgestaltung kann die Wiegung auch fortlaufend während des Mischens durchgeführt werden. Vorzugsweise handelt es sich bei den Drucksensoren um piezoresistive Drucksensoren.

Gegenstand der Verfahren ist auch ein Verfahren zum Mischen von in einem Blutbeutel befindlichem Antikoagulans mit während einer Blutentnahme in den Blutbeutel einlaufendem Frischblut, wobei der horizontale Blutbeutel in Bewegung versetzt wird, welches dadurch gekennzeichnet ist, daß der Blutbeutel mit dem flüssigen Antikoagulans und dem zufließenden Frischblut in horizontal bleibender Lage in eine in dieser Horizontalebene stattfindende kreisförmige Taumelbewegung versetzt wird.

Im folgenden wird die Erfindung anhand einer ein Ausführungsbeispiel darstellenden Zeichnung näher erläutert. Es zeigen
- Fig. 1: eine Aufsicht auf eine Vorrichtung und
- Fig. 2: einen Schnitt A-A durch den Gegenstand der Fig. 1.

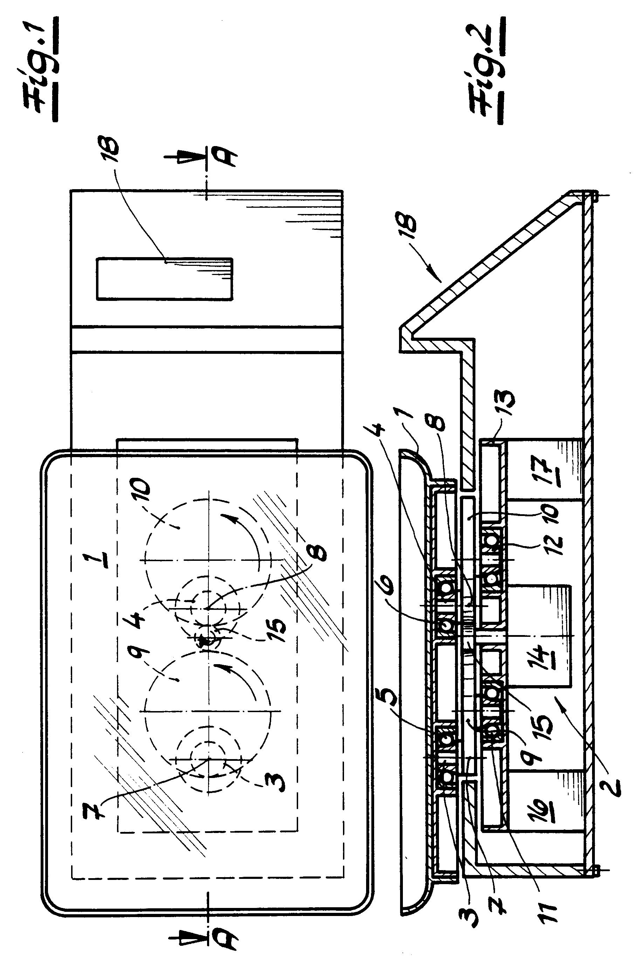

Die in den Figuren dargestellte Vorrichtung dient zum Mischen von flüssigem Antikoagulans, welches sich in einem Blutbeutel befindet, mit Frischblut, das während einer Blutentnahme in den Blutbeutel einläuft. Der Blutbeutel mit dem Antikoagulans bzw. Frischblut ist im einzelnen nicht dargestellt.

In ihrem grundsätzlichen Aufbau besteht die Vorrichtung aus einer flachen Aufnahmeschale 1 für den Blutbeutel, die von einem Antrieb 2 in Bewegung versetzbar ist. Im einzelnen ist hierzu die Anordnung so getroffen, daß die horizontal angeordnete Aufnahmeschale 1 auf zwei mit vorgegebenem Abstand voneinander angeordneten Vertikalzapfen 3, 4 mit Lagern 5,6 drehbar gelagert ist. Die beiden Vertikalzapfen 3, 4 sind mit derselben Exzentrizität auf jeweils einer um eine vertikale Achse 7, 8 drehbar gelagerten Exzenterscheibe 9, 10 befestigt. Die beiden Exzenterscheiben 9, 10 sind über Lager 11, 12 drehbar in einer plattenförmigen Lageranordnung 13 vorgesehen, an der auch der Antrieb 2 befestigt ist. Dieser Antrieb 2 besteht aus einem Elektromotor 14 mit Reibrad 15. Die beiden Exzenterscheiben 9, 10 weisen denselben Durchmeser auf und sind von dem zwischen diesen angeordneten Reibrad 15 angetrieben. Die Lageranordnung 13 ihrerseits ist auf piezoresistiven Drucksensoren 16, 17 einer Wiegeeinrichtung aufgelagert, die auf der Frontseite der Vorrichtung eine entsprechende Anzeige 18 aufweist.

Die Exzentrizität des Vertikalzapfens 3 bzw. 4 gegenüber der Drehachse 7 bzw. 8 der zugeordneten Exzenterscheibe 9 bzw. 10 soll wenigstens 20 mm ausmachen, was aus den Figuren so ohne weiteres nicht erkennbar ist, weil diese nicht maßstäblich sind. Jedenfalls sind die Exzenterscheiben 9, 10 mit wenigstens einer Umdrehung pro Sekunde angetrieben.

Zum Mischen von in einem Blutbeutel befindlichen flüssigen Antikoagulans mit während einer Blutaufnahme in den Blutbeutel einlaufendem Frischblut wird der Blutbeutel in die Aufnahmeschale 1 eingelegt und während der Blutentnahme in mehr oder weniger horizontaler Lage einer kreisförmigen Taumelbewegung ausgesetzt. Die kreisförmige Taumelbewegung wird vorteilhaft mit einem Radius von wenigstens 20 mm und mit wenigstens etwa einer Umdrehung pro Sekunde ausgeführt.

## Patentansprüche

1. Vorrichtung zum Mischen von in einem Blutbeutel befindlichem flüssigen Antikoagulans mit während einer Blutentnahme in den Blutbeutel einlaufendem Frischblut, bestehend aus einer von einem Antrieb in Bewegung versetzbaren flachen Aufnahmeschale (1) für den Blutbeutel, **dadurch gekennzeichnet**, daß die horizontal angeordnete Aufnahmeschale (1) auf zwei mit vorgegebenem Abstand voneinander angeordneten Vertikalzapfen (3, 4) drehbar gelagert ist, daß die beiden Vertikalzapfen (3, 4) mit derselben Exzentrizität auf jeweils eine um eine vertikale Achse (7, 8) drehbar gelagerten Exzenterscheibe (9, 10) befestigt sind, und daß zumindest eine der beiden Exzenterscheiben (9, 10) vom Antrieb (2) in Drehung versetzbar ist.

2. Vorrichtung nach Anspruch 1, dadurch gekennzeichnet, daß der Antrieb (2) aus einem Elektromotor (14) mit Reibrad (15) besteht.

3. Vorrichtung nach Anspruch 2, dadurch gekennzeichnet, daß die beiden, denselben Durchmesser aufweisenden Exzenterscheiben (9, 10) von dem zwischen diesen angeordneten Reibrad (15) angetrieben sind.

4. Vorrichtung nach einem der Ansprüche 1 bis 3, dadurch gekennzeichnet, daß die Exzentrizität des Vertikalzapfens (3 bzw. 4) gegenüber der Drehachse (7 bzw. 8) der zugeordneten Exzenterscheibe (9 bzw. 10) wenigstens 20 mm ausmacht.

5. Vorrichtung nach einem der Ansprüche 1 bis 4, dadurch gekennzeichnet, daß die Exzenterscheiben (9, 10) mit wenigstens einer Umdrehung pro Sekunde angetrieben sind.

6. Vorrichtung nach einem der Ansprüche 1 bis 5, dadurch gekennzeichnet, daß der Antrieb (2) und die beiden Exzenterscheiben (9, 10) an einer Lageranordnung (13) vorgesehen sind, die auf Drucksensoren (16, 17) einer Wiegeeinrichtung aufgelagert ist.

7. Vorrichtung nach Anspruch 6, gekennzeichnet durch piezoresistive Drucksensoren (16, 17).

8. Verfahren zum Mischen von in einem Blutbeutel befindlichem Antikoagulans mit während einer Blutentnahme in den Blutbeutel einlaufendem Frischblut, wobei der horizontale Blutbeutel in Bewegung versetzt wird, dadurch gekennzeichnet, daß der Blutbeutel mit dem flüssigen Antikoagulans und dem zufließenden Frischblut in horizontal bleibender Lage in eine in dieser Horizontalebene stattfindende kreisförmige Taumelbewegung versetzt wird.
